# EUROPÄISCHE PATENTSCHRIFT

(11) **EP 1 410 766 B1**
(45) Veröffentlichungstag und Bekanntmachung des Hinweises auf die Patenterteilung: **19.05.2010**
(21) Anmeldenummer: 03021574.3
(22) Anmeldetag: 24.09.2003
(51) Int. Cl.: A61B 18/22

(54) **Intelligente Therapiefaser**
Intelligent fibre for therapy
Fibre thérapeutique intelligente

(30) Priorität: 27.09.2002 DE 10245140
(43) Veröffentlichungstag der Anmeldung: 21.04.2004
(73) Patentinhaber: Dornier MedTech Laser GmbH, 82234 Wessling (DE)
(72) Erfinder: Hiereth, Werner, 82205 Gilching (DE); Austen, Jürgen, 85356 Freising (DE)
(74) Vertreter: Grünecker, Kinkeldey, Stockmair & Schwanhäusser Anwaltssozietät

(56) Entgegenhaltungen:
- EP-A- 0 473 987
- WO-A-99/15237
- DE-A- 10 009 004
- US-A- 4 722 337
- US-A- 5 681 307
- US-A- 5 742 718

## Beschreibung

Die vorliegende Erfindung bezieht sich auf ein Lasersystem mit einem Lichtleiter zur Strahlführung der erzeugten Laserstrahlung sowie auf einen Lichtleiter für Anwendungen im medizinischen Bereich, gemäß Anspruch 1 und 2, wie auch aus WO 99/15237 bekannt.

Die immer zahlreicher werdenden Anwendungsgebiete der Lasertechnologie in der Medizin führen zur Entwicklung von technisch immer mehr ausgereiften Laserkonstruktionen und entsprechenden Systemkonzepten, die die Handhabung der Lasersysteme erleichterten und verbesserten bzw. wieder neue Einsatzgebiete erschlossen.

In diesem Zusammenhang kommt dem Einsatz von flexiblen optischen Transmissionssystemen für die erzeugte Laserstrahlung eine wesentliche Bedeutung zu, denn für die Applikation der Laserstrahlung auf oder in den Therapieort muss die Entfemung zwischen dem Lasergeräteausgang und dem Patienten überbrückt werden. Die medizinischen Lasersysteme bestehen daher typischerweise aus einem stationären oder mobilen Lasergerät, einer Strahlführung, optischen Endgeräten und Zubehör für spezielle medizinische Applikationen. Für die Übertragung von sichtbarem Laserlicht und den angrenzenden Spektralbereichen von ca. 0,3 µm bis 2,1 µm werden flexible Glas- bzw. Quarzfasern verwendet. In den Spektralbereichen von 0,19 µm bis 0,3 µm (EximerLaser) und 3 µm bis 10 µm (Erbium- und CO₂-Laser) finden spezielle Lichtleiter oder Spiegelgelenkarme Anwendung.

Dabei werden besonders hohe Anforderungen an die Lichtleiter bei der Übertragung gepulster, energiereicher Laserstrahlung gestellt. Die gute Handhabbarkeit und Flexibilität dieser Transmissionssysteme ist von entscheidender Bedeutung für den Einsatz der Lasersysteme.

Die hierfür verwendeten Lichtleiter haben die unterschiedlichsten Spezifikationen bezüglich Transmissionsverhalten, maximal applizierbarer Laserleistung, Verfallsdatum bei Sterilfasern usw.. Diese Spezifikationen geben gewisse Randbedingungen hinsichtlich der Einsetzbarkeit bestimmter Lichtleitertypen in Kombination mit bestimmten Lasern oder Behandlungsparametern vor. Die Einhaltung dieser Randbedingungen wird bis dato meist über die Gebrauchsanweisung, die mit dem Laser oder dem Lichtleiter mitgeliefert wird, mit dem Anwender kommuniziert. Die Verantwortung liegt damit beim Anwender eines Lasergerätes und kann nicht durch ein Kontrollsystem im Lasergerät geprüft werden.

Die Folgen bei Nichtbeachtung oder Fehlinterpretation dieser Randbedingungen durch den Anwender sind z.B. Zerstörung der Faser, zu geringe Laserleistung am Faserende oder eine Behandlung mit einer unsterilen Faser. Das entsprechende Ergebnis kann eine erfolglose Behandlung oder unmittelbare gesundheitliche Gefährdung des Patienten sein.

Kommt es in Folge dessen zu Haftungsforderungen seitens des Anwenders wegen einer Fehlfunktion einer geschädigten Faser oder seitens des Patienten wegen gesundheitlicher Schädigung, kann im nachhinein zwischen einem Qualitätsfehler bei der Faser und einer Nichteinhaltung der Randbedingungen für die Verwendung des Lichtleiters durch den Anwender nicht mehr unterschieden werden.

Hierbei kommt Einmal-Lichtleitern für eine Kontakt- bzw. kontaktfreie Laser-Therapie eine besondere Bedeutung zu, da sie problemlos mit Endoskopen und anderen Laserführungsinstrumenten verwendet werden können und aufgrund ihrer Vorteile weit verbreitet sind. Sie sind sofort einsetzbar, sind steril verpackt und werden ab Werk mit nachvollziehbarer Qualität ausgeliefert.

Es ist daher die Aufgabe der vorliegenden Erfindung, ein Lasersystem und einen Lichtleiter anzugeben, die die Einhaltung der Randbedingungen zur Benutzung eines Lichtleiters in dem Lasersystem erleichtern und Fehlbedienungen des Lasersystems in Zusammenhang mit dem Lichtleiter unmöglich bzw. nachvollziehbar machen.

Diese Aufgabe wird in erfinderischer Weise durch den Gegenstand des Anspruchs 1 und 2 gelöst. Die vorliegende Erfindung baut auf der Erkenntnis auf, dass ein mit dem Lichtleiter fest verbundener Transponder Identifikationsdaten an das Lasergerät und das Lasergerät Parametereinstellung an den Transponder des Lichtleiters übermitteln kann, die zu einem späteren Zeitpunkt ausgewertet werden können.

Zum einen kann daher erreicht werden, dass das Lasergerät bei falscher Einstellung der Randbedingungen in Bezug auf den Lichtleiter eine Warnmeldung ausgibt bzw. Lasergeräteinstellungen aufgrund der vom Transponder übertragenen Daten automatisch vorgenommen werden. Zum anderen wird erreicht, dass zur Bewertung, ob ein Qualitätsfehler beim Lichtleiter oder ein Anwendungsfehler vorliegt, die bei der Verwendung des Lichtleiters aufgezeichneten Parametereinstellungen herangezogen werden können. Dies ist besonders wichtig für Einmal-Lichtleiter, da deren Qualität und Belastungsgrenzen entsprechend einer Therapieanwendung ausgelegt sind.

Gemäß einer bevorzugten Ausführungsform beinhaltet das Lasersystem ein Lasergerät zur Erzeugung von Laserstrahlung und einen Lichtleiter zur Strahlenführung der erzeugten Laserstrahlung. Ein Datenträger für Identitätsdaten ist mit dem Lichtleiter fest verbunden. Zusätzlich ist eine Leseeinrichtung zum Auslesen der Identitätsdaten im Lasergerät angeordnet.

Gemäß einer weiteren bevorzugten Ausführungsform beinhaltet das Lasersystem ein Lasergerät zur Erzeugung von Laserstrahlung und einen Lichtleiter zur Strahlenführung der erzeugten Laserstrahlung. Ein Datenträger für Identitätsdaten ist mit dem Lichtleiter fest verbunden. Zusätzlich ist eine Leseeinrichtung zum Auslesen der Identitätsdaten im Lasergerät angeordnet. Der Datenträger ist ein Transponder, der sowohl lesbar als auch beschreibbar ist. Hierzu verfügt die Leseeinrichtung im Lasergerät auch über eine Schreibeinrichtung zum berührungslosen Einschreiben von Daten in den Transponder. Als Identitätsdaten sind z.B. Informationen über einen Hersteller des Lichtleiters, ein Verfallsdatum des Lichtleiters, Transmission des Lichtleiters, Typenbezeichnung des Lichtleiters oder Faserdurchmesser des Lichtleiters im Transponder abgespeichert. Darüber hinaus werden zusätzlich auch Daten über eine spezifische Verwendung des Lichtleiters in Verbindung mit dem Lasergerät gespeichert. Diese Verwendungsdaten enthalten Informationen über dem Lichtleiter zugeführte Laserenergie, Anzahl der Laserpulse, Anzahl der Behandlungen mit dem Lichtleiter, Datum der Behandlung oder Identifikationsdaten des Lasergeräts. Bereits gespeicherte Verwendungsdaten sind nicht mehr löschbar, überschreibbar oder änderbar. Die Identitätsdaten und die Verwendungsdaten sind dabei verschlüsselt im Transponder abgespeichert. Der Lichtleiter ist mittels einer Befestigungseinrichtung lösbar am Lasersystem befestigt und der Datenträger in dem am Lichtleiter angebrachten Teil der Befestigungseinrichtung im Wesentlichen untrennbar angebracht. Vorzugsweise ist der Transponder in dem am Lichtleiter angebrachten Teil der Befestigungseinrichtung vergossen oder damit verschweißt oder verklebt.

Gemäß einer weiteren bevorzugten Ausführungsform wird ein Lichtleiter zur Strahlführung von Laserstrahlung vorgestellt, der mittels einer Befestigungseinrichtung lösbar an einem Lasergerät koppelbar ist und ein Datenträger für Identitätsdaten mit dem Lichtleiter fest verbunden ist.

Bei einer Alternative dieser bevorzugten Ausführungsform ist der Datenträger ein Transponder, der sowohl lesbar als auch beschreibbar ist, um zusätzlich auch Daten über eine spezifische Verwendung des Lichtleiters in Verbindung mit einem Lasergerät zu speichern. Die im Transponder gespeicherten Identitätsdaten enthalten Informationen über Hersteller des Lichtleiters, Verfallsdatum des Lichtleiters, Transmission des Lichtleiters, Typenbezeichnung des Lichtleiters oder Faserdurchmesser des Lichtleiters. Die im Transponder gespeicherten Verwendungsdaten enthalten Informationen über eine dem Lichtleiter zugeführte Laserenergie, Anzahl der dem Lichtleiter zugeführten Laserpulse, Anzahl der Behandlungen, Datum der Behandlung oder Identifikationsdaten des Lasergeräts. Die gespeicherten Verwendungsdaten sind nicht mehr löschbar und nicht überschreibbar oder änderbar. Sowohl Identitätsdaten als auch Verwendungsdaten sind verschlüsselt im Datenträger abgespeichert. Die Befestigungseinrichtung des Lichtleiters ist aus einem Material, das elektromagnetische Strahlung im Frequenzbereich eines Sende- und Empfangsbereichs des Transponders im Wesentlichen nicht abschirmt und vorzugsweise aus Kunstoff besteht. Der Lichtleiter ist mit der Befestigungseinrichtung im Wesentlichen untrennbar verbunden und der Transponder mit der Befestigungseinrichtung verschweißt, verklebt oder vergossen.

Anhand der in den beiliegenden Zeichnungen dargestellten bevorzugten Ausführungsformen wird die Erfindung im Folgenden näher erläutert. Ähnliche oder korrespondierende Einzelheiten sind in den Figuren mit den selben Bezeichnungen versehen. Es zeigen:
Fig. 1 eine schematische Darstellung eines Lasersystems gemäß einer bevorzugten Ausführungsform der vorliegenden Erfindung,
Fig. 2a und 2b eine weitere schematische Darstellung einer bevorzugten Ausführungsform gemäß der vorliegenden Erfindung,
Fig. 3 ein Flussdiagramm für den schematischen Ablauf der Datenkommunikation zwischen dem Transponder des Lichtleiters und der Lese- und Schreibeinrichtung des Lasergeräts gemäß einer bevorzugten Ausführungsform der vorliegenden Erfindung, und
Fig. 4 eine schematische Übersicht der im Transponder abgelegten Verwendungsdaten gemäß einer bevorzugten Ausführungsform der vorliegenden Erfindung.

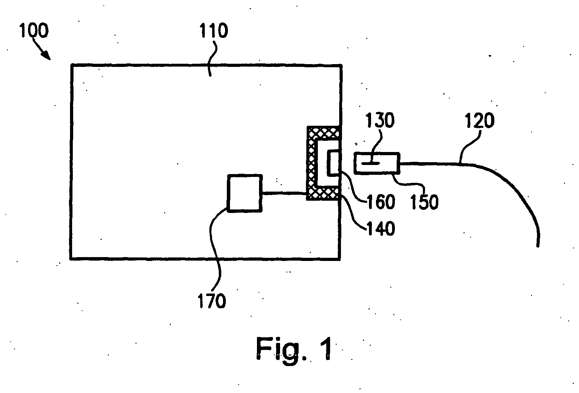

Fig. 1 zeigt anhand einer schematischen. Darstellung ein Lasersystem gemäß einer bevorzugten Ausführungsform der vorliegenden Erfindung. Das Lasersystem 100 besteht aus einem stationären oder mobilen Lasergerät 110, das eine Einrichtung zur Erzeugung von Laserstrahlung beinhaltet, an das ein flexibler Lichtleiter 120 zur Strahlführung der erzeugten Laserstrahlung koppelbar ist.

Das Lasergerät ist zur Erzeugung von intensiver Laserstrahlung mit Hochleistungslaserdioden, einer Mikrooptik zum Fokussieren des erzeugten Laserlichts sowie einer Energieversorgung ausgestattet. Alternativ ist das Lasergerät mit einem Lasermedium, einem Resonator und einer Pumpquelle sowie der entsprechenden Energieversorgung ausgestattet. In diesem Fall finden bevorzugterweise diodengepumpte Festkörperlasermedien Anwendung für die Erzeugung der intensiven Laserstrahlung.

Darüber hinaus verfügt das Lasergerät vorzugsweise über eine Kühleinrichtung sowie einen Systemkontroller, der unter anderem auch die Leistung der Laserstrahlung, die Pulsdauer sowie die Frequenz der Laserpulse regelt. Darüber hinaus sind in das Lasergerät Anzeige- und Bedieneinrichtungen integriert, über die bestimmte Anwendungsmodi sowie Systemeinstellungen ausgewählt werden können. Des Weiteren verfügt das Lasergerät über entsprechende Sicherheitseinrichtungen sowohl für den elektrischen wie auch für den optischen Bereich. Vorzugsweise besitzt der Systemkontroller entsprechende Einrichtungen, um die Steuerung und Regelung des Lasersystems über Softwareprogramme durchführen zu können. Besonders vorteilhaft ist dabei eine Ausführungsform, bei der Softwareprogramme bei einer Aktualisierung ausgetauscht werden. Bei einer weiteren Alternative der bevorzugten Ausführungsform der vorliegenden Erfindung ist in das Lasergerät eine Ausgabeeinheit für ein Protokoll der Systemeinstellungen integriert bzw. weist das Lasergerät eine Schnittstelle für eine Ausgabeeinheit auf. Des Weiteren ist in das Lasergerät eine Befestigungseinrichtung integriert, die zur festen oder lösbaren Befestigung des Lichtleiters 120 dient. Die Befestigungseinrichtung ist vorzugsweise eine Steck-, Schraub- der Bajonettverbindung, wobei der am Lasergerät befestigte Teil der Befestigungseinrichtung vorzugsweise als Buchse 160 und der am Lichtleiter befestigte Teil als Stecker 150 ausgeprägt ist. Besonders bevorzugterweise wird zur lösbaren Befestigung des Lichtleiters ein sogenannter SMA-Konnektor verwendet.

Der Lichtleiter kann aus einer oder mehreren Plastik-, Glas- oder Quarzglasfasern bestehen. Je nach Wellenlänge der erzeugten Laserstrahlung werden auch dotierte Quarzglasfasern verwendet. Der Lichtleiter ist dafür ausgelegt, hohe Lichtleistungen möglichst verlustfrei transportieren zu können. Er verfügt aus Sicherheitsgründen vorzugsweise über eine entsprechende Ummantelung, die die Faser vor entsprechender mechanischer Belastung und im Fall eines Bruchs der Faser vor einem Austreten der Laserstrahlung schützen. Besonders bevorzugterweise handelt es sich bei dem Lichtleiter 120 um einen sogenannten Einmal-Lichtleiter. Dies ist eine steril verpackte Therapiefaser zur einmaligen Verwendung.

Der Stecker 150 ist vorzugsweise aus einem Material, das elektromagnetische Strahlung im Frequenzbereich eines Sende- und Empfangsbereichs des Transponders im Wesentlichen nicht abschirmt und vorzugsweise aus Kunststoff. Der Stecker und der Lichtleiter sind im Wesentlichen untrennbar miteinander verbunden. In den Stecker 150 ist ein Transponder eingebracht. Auf diese Art sind die Glasfaser, die Buchse der Befestigungseinrichtung und der Transponder fest miteinander verbunden. Bevorzugterweise ist der Transponder fest in der Buchse verschweißt, verklebt oder vergossen, so dass er nicht entfernt werden kann.

Der Transponder enthält einen Lese/Schreibe-Speicher zur Aufnahme sämtlicher relevanter Informationen, die bei der Herstellung des Therapielichtleiters und während der Verwendung am Lasergerät generiert werden. Das Lasergerät wird hierzu mit einer Platine zum Lesen und Beschreiben des Lichtleitertransponders ausgerüstet. Die Datenübertragung erfolgt drahtlos im RF 3,5 KHz-Band mittels Antenne. Wie bereits oben erwähnt, werden in dem Lasersystem Daten auf einem elektronischen Datenträger gespeichert. Bevorzugterweise werden hierfür sog. RFID-Systeme (radio-frequency identification) verwendet. An dem zu identifizierenden Lichtleiter sind sog. Transponder angebracht. Die Energieversorgung des Transponders sowie der Datenaustausch zwischen Transponder und Lesegerät erfolgt jedoch nicht durch galvanischen Kontakt, sondern berührungslos mittels magnetischer oder elektromagnetischer Felder.

Das RFID-System besteht immer aus zwei Komponenten, dem oben erwähnten Transponder 130, der am zu identifizierenden Lichtleiter angebracht ist und einem Lesegerät 170 mit Antenneneinheit 140, das je nach Ausführung sowohl als Lese- als auch als Schreib/Lese-Einrichtung ausgeführt ist. Das Lesegerät ist alternativ mit einem lokalen Computernetzwerk gekoppelt. Das Lesegerät ist vorzugsweise mit dem Systemkontroller des Lasergerätes verbunden.

Das Lesegerät besteht vorzugsweise im wesentlichen aus einer Steuereinheit und einem Hochfrequenz-Interface (HF-Interface). Grundsätzliche Aufgabe des Lesegeräts ist die Aktivierung des Transponders, der Aufbau einer Kommunikation und der Transport der Daten zwischen einer Applikationssoftware des Systemkontrollers des Lasergerätes und dem kontaktlosen Datenträger. Für die beiden Datenflussrichtungen von und zum Transponder stehen zwei getrennte Signalzüge innerhalb des HF-Interfaces zur Verfügung. Daten, die zum Transponder übertragen werden, durchlaufen den Senderzweig. Hingegen werden Daten, die vom Transponder empfangen werden, im Empfängerzweig aufbereitet.

In dem RFID-System findet sowohl ein Austausch von Daten als auch von Energie statt. Innerhalb des Transponders ist zwischen Speicher und Sende/Empfangsantenne ein Wandler geschaltet, der die analogen Signale der Antenne in für den Speicher verwertbare, digitale Signale umsetzt. Der komplette Ablauf wird dabei durch eine Steuerlogik in einem Mikrochip des Transponders überwacht.

Außerhalb des Ansprechbereichs eines Lesegeräts verhält sich der Transponder passiv, da er in der Regel keine eigene Spannungsversorgung besitzt. Erst innerhalb des Ansprechbereichs wird er durch das Lesegerät aktiviert, indem zum Betrieb des Transponders benötigte Energie über eine Sende-/Empfangsantenne übertragen wird. Bevorzugterweise ist der Transponder programmierbar und ohne Batterie (passiv). Alternativ finden auch fest programmierte Transponder mit oder ohne Batterie Anwendung oder sog. semipassive Transponder, bei denen der Mikrochip von einer Batterie gespeist lediglich für die Datenübertragung das Feld des Lesegeräts induktiv genutzt wird.

In dem Lasersystem werden je nach Anwendungszweck RFID-Systeme mit verschiedenen Reichweiten verwendet. Zum einen werden Close-Coupling-Systeme, also als eng koppelnde Systeme mit sehr kleiner Reichweite von bis zu ca. 0,01 m eingesetzt. Der Transponder muss hierbei entweder in ein Lesegerät eingesteckt oder auf einer dafür vorgesehenen Oberfläche positioniert werden. Zur Übertragung kommen beliebige Frequenzen bis zu 30 MHz zum Einsatz. Durch die enge Kopplung zwischen Datenträger und Lesegerät werden große Energiemengen für Anwendungen bereit gestellt, die entsprechende Sicherheitsanforderungen stellen, jedoch keine große Reichweite erfordern.

Alternativ werden Remote-Coupling-Systeme verwendet, die Reichweiten von bis zu 1 m ermöglichen. Sie haben alle die induktive Kopplung zwischen Lesegerät und Transponder gemeinsam. Als Sendefrequenzen werden typischerweise Frequenzen unter 135 KHz sowie der Bereich um 13,56 MHz verwendet.

Bei einer weiteren Alternative werden Long-Range-Systeme verwendet, bei denen Reichweiten von deutlich über 10 m möglich sind. Dabei reicht die übertragene Energie in keinem Fall aus, um den Transponder mit ausreichend Energie zum Betrieb des Mikrochips zu versorgen. Eine Stützbatterie stellt dann ausschließlich die Energie für den Mikrochip und die Erhaltung der gespeicherten Daten bereit (semiaktive Energieversorgung). Die Sendefrequenzen liegen hier typischerweise im Mikrowellenbereich (2,45 - 5,8 GHz).

Bevorzugterweise wird als kostengünstige Alternative ein Read-Only Transponder verwendet. Sobald der Read-Only Transponder in den Ansprechbereich des Lesegeräts geräts 170 gerät, beginnt die Ausgabe einer bestimmten Kennung (Seriennummer) des Transponders, die während der Mikrochip-Produktion aufgebracht wurde. Diese Kennung sowie weitere Daten sind werkseitig unveränderbar in den Speicher des Transponders eingeschrieben.

Besonders bevorzugterweise wird als weitere Alternative ein Transponder verwendet, der durch das Lesegerät mehrmals mit Daten beschreibbar und mit einem Read/Write-Speicher ausgerüstet ist. Die Datenübertragung erfolgt typischerweise blockweise. Das heißt, dass eine definierte Anzahl von Bytes zu einem Block zusammengefasst wird, die dann nur als Ganzes gelesen oder beschrieben wird. Diese Blockstruktur ermöglicht dabei eine einfachere Adressierung im Mikrochip und durch das Lesegerät. Die Speichergrößen des Read/Write-Transponders variieren je nach Anwendung typischerweise zwischen 1 Byte und 64 kByte.

Für Anwendungsfälle der Therapiefaser, bei denen eine mehrfache Wiederbeschreibung nicht notwendig ist, wird alternativ ein Write-Once-Transponder verwendet, der nur einmal beschreibbar ist.

Um die gespeicherten Daten gegen unerwünschten Zugriff zu schützen, wird bevorzugterweise im Mikrochip eine sog. Kryptoeinheit integriert, die zur Identifizierung, Datenverschlüsselung und Schlüsselverwaltung verwendet wird. Bevorzugterweise bietet die Kroptoeinheit einen Passwortschutz und einen werkseitig eingestellten 64-Bit Schlüssel.

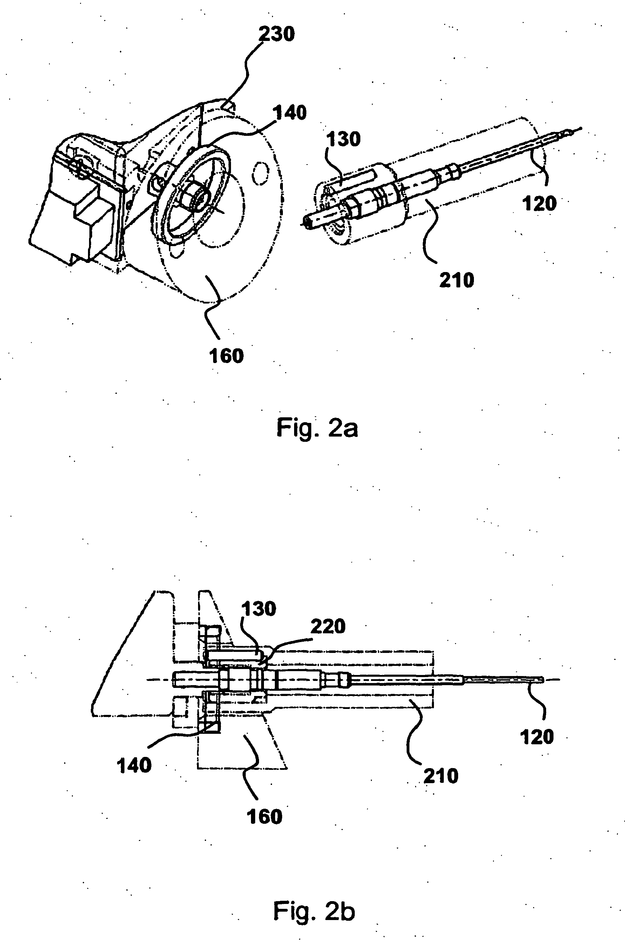

Fig. 2a und 2b zeigen anhand weiterer schematischer Darstellung eine besonders bevorzugte Ausführungsform der vorliegenden Erfindung. Der Lichtleiter 120 ist mit dem Stecker und dem Steckergehäuse 210 fest verbunden. Bevorzugterweise ist der Lichtleiter im Wesentlichen unlösbar mit dem Stecker verbunden. Der Lichtleiter ist dabei durch den Stecker durchgeführt und am offenen Ende des Steckers herausgeführt, so dass an diesem Ende die erzeugte Laserstrahlung in den Lichtleiter einkoppelbar ist.

In den Innenraum des Steckers ist der Transponder 130 bevorzugterweise mit Vergussmasse 220 eingegossen, so dass er im Wesentlichen untrennbar mit dem Lichtleiter und dem Stecker verbunden ist. Alternativ ist der Transponder in das Steckergehäuse eingeschweißt oder mit dem Steckergehäuse verklebt. Alternativ sind weitere Befestigungsmöglichkeiten vorgesehen, die es ermöglichen, den Transponder unlösbar mit dem Lichtleiter und dem Stecker zu verbinden bzw. dafür sorgen, dass eine zerstörungsfreie Entfernung des Transponders aus dem Stecker nicht möglich ist. Auf diese Art und Weise wird sichergestellt, dass der Transponder immer mit dem Lichtleiter gekoppelt ist und die im Transponder gespeicherten Identifikations- und Verwendungsdaten immer mit dem Lichtleiter mitgeführt werden. Dies ermöglicht zum einen, dass eine Fehlbedienung des Lasergerätes in Zusammenhang mit dem Lichtleiter verhindert werden kann, und zum anderen wird sichergestellt, dass die Historie der Verwendung des Lichtleiters jederzeit nachvollziehbar ist.

Im Lasergerät ist das Gegenstück 160 für die Steckverbindung 150 in der Gehäusewand 230 des Lasergerätes angebracht. Wie bereits oben erwähnt, werden alternativ Schraubverbindungen oder andere Befestigungseinrichtungen verwendet. Besonders bevorzugterweise finden hier sog. SMA-Konnektoren Verwendung.

Je nach oben erwähnten, verwendetem Transpondertyp ist in dem Lasergerät eine entsprechende Sende- und Empfangseinrichtung 140/170 angeordnet. Bevorzugterweise handelt es sich hier um eine Antenne 140, die in der Nähe der Steck- oder Schraubverbindung 150/160 angebracht ist. Auf diese Art und Weise wird sichergestellt, dass der Empfang des RFID-Systems entsprechend zuverlässig funktioniert und ein ausreichend guter Signal-Rauschverhältnis gewährleistet ist. Sende- und Empfangseinrichtung 140/170 sowie der Transponder 130 sind so angeordnet, dass sie durch Komponenten des Lasersystems im Wesentlichen nicht abgeschirmt werden, so dass ein entsprechend guter Empfang in dem RFID-System zu gewährleistet ist, wie in Fig. 2b ersichtlich.

Die Antenne 140 ist mit einem Hochfrequenz-Interface gekoppelt, das wiederum mit einer Steuereinheit verbunden ist. Von der Steuereinheit werden Empfangs- und Sendedaten mit dem Hochfrequenz-Interface ausgetauscht. Die Steuereinheit ist bevorzugterweise mit dem Systemkontroller des Lasergerätes verbunden. Dadurch wird es ermöglicht, dass die aus dem Transponder ausgelesenen Lichtleiterdaten über das Hochfrequenz-Interface ausgelesen und über die Steuereinheit an den Systemkontroller weitergeleitet werden können. Der Systemkontroller kann entweder durch Anweisungen auf der Anzeigeeinrichtung auf notwendige Systemeinstellungen hinweisen oder automatisch entsprechende Systemeinstellungen vornehmen, wodurch eine Fehlbedienung des Lasergeräts mit dem verwendeten Lichtleiter minimiert wird. Dies betrifft typischerweise zum einen Einstellungen der maximalen Pulsenergie bzw. -dauer sowie die maximal mögliche Anzahl von Laserpulsen, die über den Lichtleiter zu einer Anwendestelle geführt werden: Darüber hinaus wird alternativ registriert, ob es sich bei dem Lichtleiter um einen mehrfach verwendbaren Lichtleiter handelt oder ob es sich um einen einmal verwendbaren Lichtleiter handelt. Für den letzteren Fall ist alternativ vorgesehen, bei der Verwendung von Einmaltherapiefasern entsprechende Anwendungsdaten zuerst aus dem mit dem Einmal-Lichtleiter gekoppelten Transponder auszulesen, auszuwerten und für den Fall, dass die Einmaltherapiefasern bereits verwendet wurde, eine entsprechende Warnmeldung an der Anzeigeeinrichtung anzuzeigen bzw. die Emission eines Laserpulses über den Lichtleiter zu unterbinden.

In einer alternativen Ausführungsform ist das RFID-System an dem zum Lasergerät abgewandten Ende des Lichtleiters angebracht, vor allem wenn es sich hierbei um einen Lichtleiter handelt, an dessen Ende ein Stecker/Griffstückkombination für einen sog. Applikator vorgesehen ist. Hierbei erfolgt das Auslesen und Einschreiben der Daten über eine in dem Griffstück angebrachte Antenne sowie Elektronik. Alternativ kann Sende- und Empfangseinheit des RFID-Systems auch im Lasergerät direkt angebracht sein, sofern ein Remote-Coupling-System mit einer Reichweite bis zu 1 m oder ein sog. Long-Range-System mit einer größeren Reichweite verwendet wird.

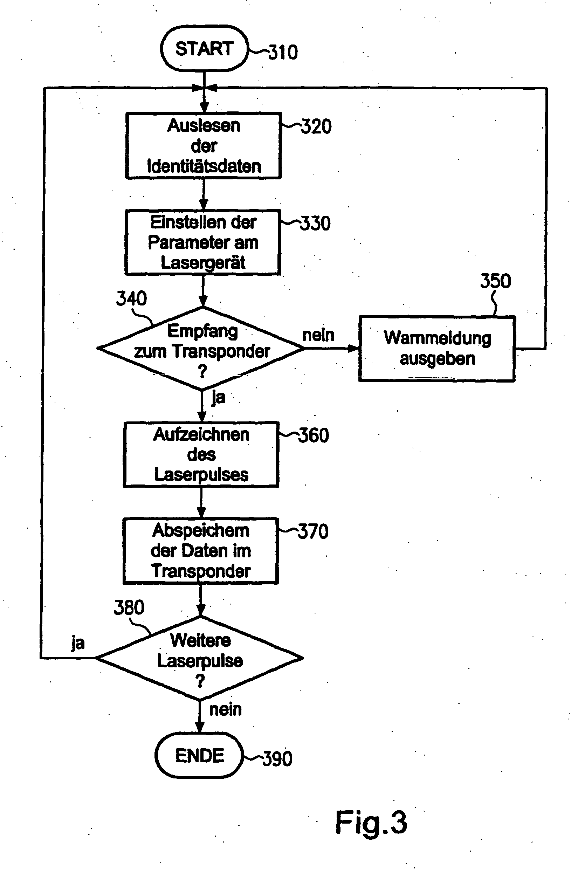

Fig. 3 zeigt ein Flussdiagramm für den schematischen Ablauf der Datenkommunikation zwischen Sende- und Empfangseinrichtung des Lasergeräts bzw. des oben erwähnten Handstücks und den mit dem Lichtleiter verbundenen Transponder gemäß der bevorzugten Ausführungsform der vorliegenden Erfindung. In Schritt 310 initiiert entweder der Systemcontroller des Lasergeräts oder die Steuereinheit den Start von Programmroutinen.

In Schritt 320 werden die Identitätsdaten aus dem Transponder ausgelesen. Ist das Auslesen der Identitätsdaten nicht möglich, wird eine entsprechende Warnmeldung auf der Anzeigeeinrichtung angezeigt bzw. wird die Emission von Laserpulsen unterbunden. Alternativ werden in Schritt 320 zusätzlich die Anwendungsdaten aus dem Transponder ausgelesen. Für den Fall, dass ein Einmal-Lichtleiter vorliegt, wird überprüft, ob entsprechende Anwendungsdaten bereits in dem Transponder hinterlegt sind bzw. ob der Einmal-Lichtleiter bereits verwendet wurde und entsprechende Daten in dem Transponder abgespeichert wurden. Für diesen Fall wird eine entsprechende Warnmeldung auf der Anzeigeeinrichtung dargestellt bzw. die Emission von Laserpulsen unterbunden. Für den Fall, dass ein mehrfach verwendbarer Lichtleiter vorliegt, werden die Anwendungsdaten ausgelesen und überprüft, ob die über den Lichtleiter emittierte Laserleistung einen spezifischen Grenzwert überschritten hat bzw. die für die Garantie der einwandfreien Funktion des Lichtleiters maximal mögliche Anzahl von Verwendungen noch nicht überschritten ist. Für den Fall, dass einer der Werte überschritten ist, wird, wie bereits oben erwähnt, eine entsprechende Warnmeldung auf der Anzeigeeinrichtung dargestellt bzw. die Emission von Laserpulsen unterbunden.

In Schritt 330 werden die entsprechenden Identitätsdäten über die Steuereinheit des Hochfrequenz-Interfaces an den Systemkontroller des Lasergeräts weitergeleitet. Diese Identitätsdaten enthalten vorzugsweise Informationen über den Hersteller, das Verfallsdatum, eine durchschnittliche Transmissionsleistung, eine maximale Transmissionsleistung, die Typenbezeichnung oder einen Faserdurchmesser des Lichtleiters. Darüber hinaus können weitere Daten zur Identifizierung des Lichtleiters wie Produktionsnummer, Losnummer, Fertigungsdatum oder ähnliches in dem Transponder abgespeichert sein.

Entsprechend diesen Daten nimmt der Systemkontroller, wie bereits erwähnt, im Lasergerät entsprechende Systemeinstellungen vor, d.h. es wird die Laserleistung, die Pulsdauer oder die maximal mögliche Anzahl von Laserpulsen automatisch eingestellt. Alternativ ist vorgesehen, dass bei manueller Bedienung des Lasergeräts beim Einstellen falscher Parameter der Systemkontroller über die Anzeigeeinrichtung entsprechende Warnmeldungen bzw. Korrekturvorschläge ausgibt.

Auf diese Art und Weise wird sichergestellt, dass eine Fehlbedienung des Lasergerätes in Zusammenhang mit dem Lichtleiter verhindert wird. Das Risiko, Laserenergien und Laserpulsdauern einzustellen, die zur Zerstörung des Lichtleiters bzw. zu einer Fehlbehandlung führen würden, wird dadurch minimiert.

In Schritt 340 wird der Empfang des HF-Interfaces zum Transponder überprüft. Auf diese Art und Weise wird sichergestellt, dass entsprechende Anwendungsdaten, wie z.B.

Laserpulsenergie und Laserpulsdauer auch in den Transponder eingeschrieben werden können. Ist kein Empfang zum Transponder 130 möglich, wird in Schritt 350 eine entsprechende Warnmeldung über die Anzeigeeinrichtung dargestellt. Der Ablauf der Steuerung beginnt dann wieder bei Schritt 320 mit dem Auslesen von Identitätsdaten aus dem Transponder. Ist ein entsprechender sicherer Empfang zum Transponder sichergestellt, wird mit Schritt 360 fortgefahren.

In Schritt 360 wird über den Systemkontroller die entsprechende Systemeinstellung aufgezeichnet und an die Steuereinheit des Hochfrequenz-Interfaces weitergegeben. In Schritt 370 werden die vom Systemkontroller ermittelten Anwendungsdaten an den Transponder weitergeleitet und eingeschrieben. In Schritt 380 überprüft der Systemkontroller oder die Steuerung des Hochfrequenz-Interfaces, ob weitere Laserpulse für die Laseranwendung emittiert werden. Für den Fall, dass weitere Laserpulse für die Laseranwendung emittiert werden, fährt die Steuerung mit Schritt 320 fort. Anderenfalls wird der Steuerungsprozess mit Schritt 390 beendet.

Alternativ wird in Schritt 320 aus dem Transponder zusätzlich eine Herstelleridentifikation des Lichtleiterherstellers ausgelesen und ausgewertet, um zu überprüfen, ob der Lichtleiter von einem autorisierten Hersteller gefertigt wurde.

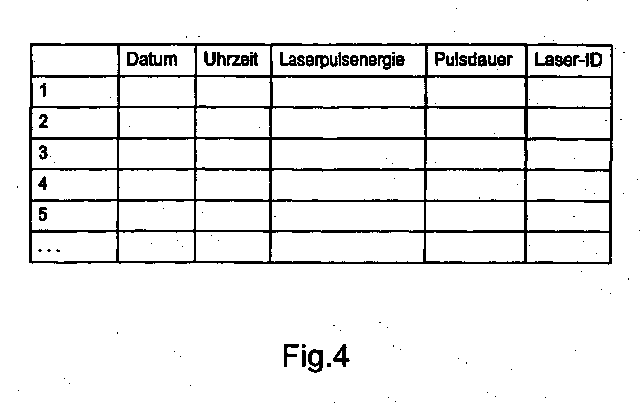

Fig. 4 zeigt eine schematische Darstellung einer Übersicht der im Transponder gespeicherten Verwendungsdaten gemäß einer bevorzugten Ausführungsform der vorliegenden Erfindung. Der Systemkontroller ermittelt ein entsprechendes Datum sowie Uhrzeit der Anwendung sowie die entsprechende Laserpulsenergie und Laserpulsdauer. Diese Informationen werden zusammen mit einer Identifikationsnummer des Lasergerätes über die Steuereinheit des HF-Interfaces an den Transponder übertragen. Dabei wird jeder einzelne Laserpuls, der über den Lichtleiter vom Lasergerät emittiert wurde, wie bereits oben erwähnt, in dem Transponder aufgezeichnet und mit einer fortlaufenden Nummer versehen.

Dies ermöglicht es, die Historie der Verwendung des Lichtleiters nachzuvollziehen. Hierzu wird der Lichtleiter mit einer entsprechenden Auswerteeinrichtung verbunden, die die entsprechenden im Transponder gespeicherten Identitätsdaten und Verwendungsdaten ausliest, entschlüsselt und auswertet. Bevorzugterweise sind die Daten in dem Transponder über die oben erwähnte Kryptoeinheit verschlüsselt abgespeichert, um sie vor Manipulation oder Fälschung zu sichern. Die Daten in dem Transponder sind nicht löschbar bzw. nicht überschreibbar oder nicht änderbar abgelegt. Auf diese Art und Weise wird sichergestellt, dass die in dem Transponder abgelegten Daten im Wesentlichen unverfälscht alle Verwendungen des Lichtleiters wiedergeben. Damit ist es möglich, im Fall einer Beschädigung des Lichtleiters nachzuvollziehen, inwiefern eine Fehlbedienung des Lasergeräts bzw. ein Nichteinhalten der Randbedingungen zur Bedienung des Lichtleiters ursächlich ist. Auf diese Art und Weise wird eine Untersuchung, ob ein Qualitätsfehler oder ein Nichteinhalten der Randbedingungen zur Anwendung des Lichtleiters vorliegt, wesentlich erleichtert und ein deutlicher Sicherheitsvorteil für den Hersteller von Therapiefasern, besonders für Einmal-Therapiefasern, geschaffen.

Die vorliegende Erfindung ist nicht auf die aufgezählten bevorzugten Ausführungsformen beschränkt, sondern erstreckt sich auch auf die Kombination aller bevorzugten Ausführungsformen.

## Patentansprüche

1. Medizinisches Lasersystem (100), umfassend:
ein Lasergerät (110) zur Erzeugung von Laserstrahlung;
einen Lichtleiter (120) zur Strahlführung der erzeugten Laserstrahlung, wobei der Lichtleiter mittels einer Befestigungseinrichtung lösbar am Lasergerät befestigt ist;
einen Transponder, wobei der Transponder derart mit dem Lichtleiter verbunden ist, dass eine zerstörungsfreie Entfernung des Transponders von dem Lichtleiter nicht möglich ist; und
eine Übertragungseinrichtung zum berührungslosen Übertragen von Daten betreffend eine spezifische Verwendung des Lichtleiters in Verbindung mit dem Lasergerät an den Transponder,
**dadurch gekennzeichnet, daß** der Transponder ausgebildet ist, die Daten unveränderbar zu speichern.

2. Einmal-Lichtleiter (120) zur Strahlführung von Laserstrahlung, umfassend einen Transponder, wobei der Lichtleiter mittels einer Befestigungseinrichtung lösbar an ein medizinisches Lasergerät koppelbar ist, der Transponder derart mit dem Lichtleiter verbunden ist, dass eine zerstörungsfreie Entfernung des Transponders von dem Lichtleiter nicht möglich ist, und der Transponder ausgebildet ist, Daten betreffend eine spezifische Verwendung des Lichtleiters in Verbindung mit dem Lasergerät zu empfangen
**dadurch gekennzeichnet, daß** der Transponder ebenfalls ausgebildet ist, diese Daten unveränderbar zu speichern.

## Claims

1. A medical laser system (100), comprising:
a laser device (110) for the generation of laser radiation;
a light guide (120) for guiding the generated laser radiation, wherein the light guide is attached in a releasable manner to the laser device by means of an attachment apparatus;
a transponder, wherein the transponder is connected to the light guide such that it is not possible to remove the transponder from the light guide without destroying the transponder; and
a transmission apparatus for the contact-free transmission of data to the transponder, said data relating to a specific use of the light guide in connection with the laser device,
**characterised in that** the transponder is designed to save the data such that they cannot be changed.

2. A single use light guide (120) for guiding laser radiation, comprising a transponder, wherein the light guide may be coupled in a releasable manner to a medical laser device by means of an attachment apparatus, the transponder is connected to the light guide such that it is not possible to remove the transponder from the light guide without destroying the transponder, and the transponder is designed to receive data with regard to a specific use of the light guide in connection with the laser device, **characterised in that** the transponder is also designed to save these data such that they cannot be changed.

## Revendications

1. Système laser (100) médical, comportant :
- un appareil à laser (110) destiné à générer un rayon laser ;
- un conducteur optique (120) pour le guidage du rayon laser généré, ledit conducteur optique étant fixé de manière amovible sur l'appareil à laser au moyen d'un dispositif de fixation ;
- un transpondeur, ledit transpondeur étant relié au conducteur optique de telle sorte qu'un détachement sans destruction du transpondeur du conducteur optique n'est pas possible ; et
- un dispositif de transmission pour la transmission sans contact vers le transpondeur de données concernant une utilisation spécifique du conducteur optique en liaison avec l'appareil à laser,
**caractérisé en ce que** le transpondeur est configuré pour stocker en mémoire les données sans possibilité de les modifier.

2. Conducteur optique à usage unique (120) pour le guidage d'un rayon laser, comportant un transpondeur, ledit conducteur optique pouvant être couplé de manière amovible à un appareil à laser médical au moyen d'un dispositif de fixation, le transpondeur étant relié au conducteur optique de telle sorte qu'un détachement sans destruction du transpondeur du conducteur optique n'est pas possible, et le transpondeur étant configuré pour recevoir des données concernant une utilisation spécifique du conducteur optique en liaison avec l'appareil à laser **caractérisé en ce que** le transpondeur est également configuré pour stocker en mémoire ces données sans possibilité de les modifier.
